# EUROPÄISCHE PATENTSCHRIFT

(11) **EP 1 441 807 B1**
(45) Veröffentlichungstag und Bekanntmachung des Hinweises auf die Patenterteilung: **25.03.2015**
(21) Anmeldenummer: 02802652.4
(22) Anmeldetag: 06.11.2002
(51) Int. Cl.: A61N 1/36

(54) **RETINAIMPLANTAT MIT VERBESSERTEN IMPLANTATIONS- UND NUTZUNGSEIGENSCHAFTEN**
RETINA IMPLANT WITH IMPROVED IMPLANTATION AND UTILISATION PROPERTIES
IMPLANT RETINIEN PRESENTANT DE MEILLEURES PROPRIETES D'IMPLANTATION ET D'UTILISATION

(30) Priorität: 09.11.2001 DE 10154700
(43) Veröffentlichungstag der Anmeldung: 04.08.2004
(73) Patentinhaber: Pixium Vision SA, 75012 Paris (FR)
(72) Erfinder: ECKMILLER, Rolf, 41460 Neuss (DE)
(74) Vertreter: Graf von Stosch, Andreas
(86) Internationale Anmeldenummer: PCT/EP2002/012387
(87) Internationale Veröffentlichungsnummer: WO 2003/039661

(56) Entgegenhaltungen:
- DE-A- 2 948 266
- US-A- 4 628 933
- US-A- 5 935 155
- LEONHARDT M L ET AL: "ELECTRONIC DESIGN OF A PHOSPHENE VISUAL PROSTHESIS" PROCEEDINGS OF THE SAN DIEGO BIOMEDICAL SYMPOSIUM, XX, XX, Bd. 12, 31. Januar 1973 (1973-01-31), Seiten 247-259, XP008011177

## Beschreibung

Die vorliegende Erfindung betrifft ein Retinaimplantat mit den Merkmalen des Oberbegriffs des Anspruchs 1.

Ein gattungsgemäßes Implantat ist aus der US 5,935,155 bekannt. In dieser Druckschrift wird vorgeschlagen, eine innerhalb der Glaskörperkammer positionierte Funktionseinheit (IGF) über eine drahtlose Kopplung zweier Spulen mit einer extern positionierten Funktionseinheiten (EPF) zu verbinden. Die Spule der internen Funktionseinheit ist dabei in der Linse hinter der Iris angeordnet.

Es ist neben dem chirurgischen Glaskörpereingriff zur Implantation der innerhalb der Glaskörperkammer positionierten Funktionseinheit (IGF) ein zweiter chirurgischer Eingriff im vorderen Augenabschnitt zur Implantation der Spule oder einer einer außerhalb der Glaskörperkammer positionierten zweiten Funktionseinheit (AGF) (insbesondere in der Linsenkapsel anstelle der vorher zu entfernenden Intraokularlinse (IOL)) erforderlich.

Die insbesondere im Falle verschiedener Retinaimplantate vorgesehene, in der Linsenkapsel implantierte zweite Funktionseinheit (AGF) ist mechanisch mit der IGF über ein Mikrokabel verbunden und kann in gegenwärtig verfügbaren Realisierungen nicht zwischenzeitlich getrennt und wieder geschlossen werden. Jedoch sind hierfür technische Lösungen verfügbar. Diese mechanische Verbindung erschwert die chirurgischen Eingriffe erheblich, da bei der Implantation der AGF im Linsenkapselsack zusätzlich eine Öffnung in der Linsenkapselwand hergestellt und IGF mit Mikrokabelverbindung durch diese Öffnung in die Glaskörperkammer transportiert werden muss. Hierdurch entstehen zusätzliche Risikofaktoren, wie: mechanische Destabilisierung des Linsenkapselsacks durch die zusätzliche Öffnung; mechanische Belastung der Implantatkomponenten einschließlich des Mikrokabels; Verlängerung der Implantationsdauer; Erhöhung des Risikos von späteren pathologischen Gewebeveränderungen, die die Implantatfunktion bzw. weitere chirurgische Eingriffe (wie z. B. Re-Explantationen) erschweren oder unmöglich machen können.

Die Positionierung von AGF in der vorderen Augenkammer schränkt den optischen Zugang zur Retina und zum IGF deutlich ein. Dies kann sich nachteilig auswirken sowohl auf die Funktion des Implantatsystems sowie das Sehvermögen als auch die medizinische Inspektionsfähigkeit der Glaskörperkammer.

Außerhalb des Körpers befindliche, extern positionierte Funktionseinheiten (EPF) sind im normalen Gesichtsfeld des Auges an Stelle eines Brillenglases oder einer Kontaktlinse unmittelbar vor dem Auge positioniert und verhindern damit ein noch möglicherweise vorhandenes partielles Sehvermögen (z. B. bei Betroffenen mit Makuladegeneration und noch vorhandener extrafovealer Sehfähigkeit).

Mit zunehmendem Signalverarbeitungsaufwand insbesondere im Fall von Retinaimplantaten bei Erhöhung der Zahl der Mikrokontakte nehmen Masse und Energiebedarf der Mikroelektronikkomponenten deutlich zu, so dass hier rasch eine Grenze der intraokular implantierbaren Funktionseinheiten erreicht wird und dadurch die erwünschte Funktionsqualität des Implantats wesentlich eingeschränkt wird.

Weitere gattungsmäßige Implantate sind aus den Druckschriften US 4,628,933, DE 29 48 266 und Leonhardt, Michael L., David A. Hodges, and Elwin Marg. "Electronic design of a phosphene visual prosthesis." In San Diego Biomedical Symposium (Feb. 1973). Conference Proc, pp. 247-259. 1973 bekannt.

Es ist deshalb Aufgabe der vorliegenden Erfindung, ein Retinaimplantat zu schaffen, bei dem die Verbindung zwischen der internen Funktionseinheit IGF und wenigstens einer externen Funktionseinheit (AGF, EPF) nicht den optischen Pfad zwischen der Linse und dem extrafovealen Bereich der Retina unterbricht und bei dem diese Verbindung drahtlos oder während der Implantation mechanisch trennbar ist.

Diese Aufgabe wird von einem Retinaimplantat mit den Merkmalen des Anspruchs 1 gelöst. Weil der Signalweg durch die Sklera des Auges innerhalb der durch die Conjunctiva begrenzten Augenhöhle verläuft, bleibt der optische Weg von der Linse zu der Retina außerhalb des fovealen Bereich frei. Die Trennbarkeit des Signalweges ermöglicht die separate Implantierbarkeit der innerhalb des Auges implantierten Komponente und der außerhalb des Auges in der Augenhöhle implantierten Komponente.

Vorteilhafte Ausführungsformen sind in den abhängigen Ansprüchen angegeben.

Im folgenden wird ein Ausführungsbeispiel der vorliegenden Erfindung anhand der Zeichnung beschrieben. Es zeigen:
- Fig. 1:: ein Implantat mit einer drahtlosen induktiven Verbindung zwischen der internen Funktionseinheit und einer außerhalb des Auges implantierten zweiten Funktionseinheit;
- Fig. 2:: ein Implantat ahnlich Figur 1, das nicht Teil der Erfindung ist, mit einer trennbaren Steckverbindung zwischen der internen Funktionseinheit und einer außerhalb des Auges implantierten zweiten Funktionseinheit; sowie

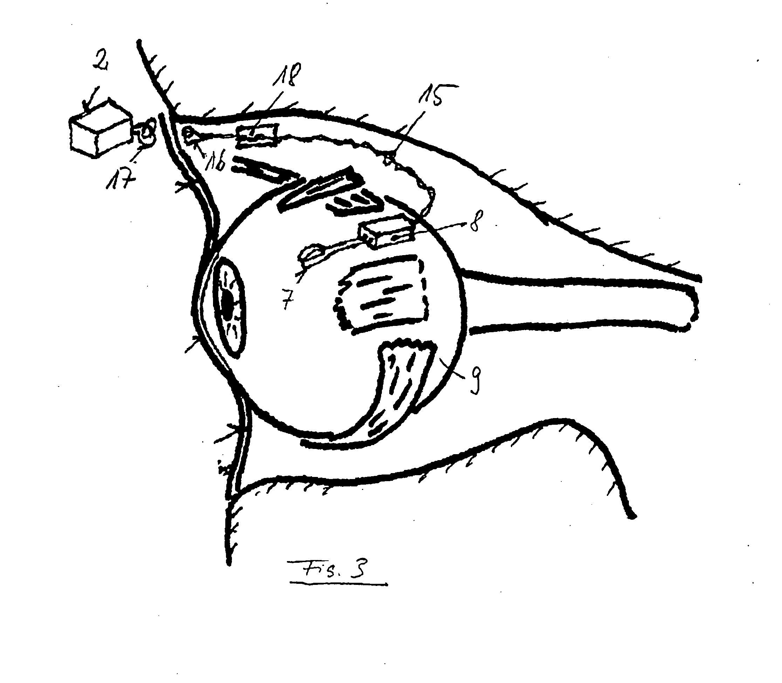

- Fig. 3:: eine perspektivische Darstellung des Implantats gemäß Figur 1.

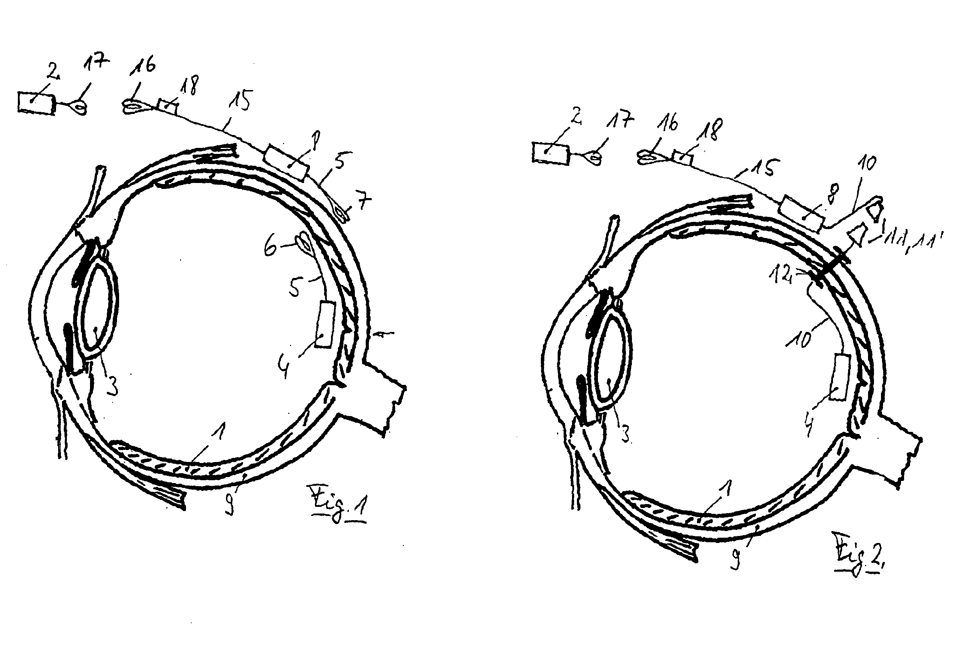

In der Figur 1 ist ein Retinaimplantat für Patienten mit einer Degenerationserkrankung der Netzhaut 1 dargestellt, bei dem die außerhalb des Körpers vorhandene Funktionseinheit (EPF) 2 so im Kopfbereich (z. B. seitlich an einem Brillengestell mit normaler Brillenglasfunktion) positioniert wird, dass der optische Strahlengang zwischen Sehobjekten und der Retina 1 weder durch Funktionseinheiten direkt vor dem Auge noch durch Funktionseinheiten im vorderen Augenabschnitt einschließlich der Intraokularlinse (IOL) 3 beeinträchtigt wird und dass auf diese Weise insbesondere Patienten ihre eventuell noch vorhandene Restsehfähigkeit (z. B. im extrafovealen Gesichtsfeld bei Makuladegeneration) zusätzlich zur Implantatsfunktion nutzen können.

Bei einem erfindungsgemäßen Retinaimplantat wird die innerhalb der Glaskörperkammer positionierte Funktionseinheit IGF 4 als Mikrokontaktfolie mit einer zugehörigen Mikroelektronik, einem Mikrokabel 5 und mindestens einer Spule 6 ausgeführt und in geeigneter Weise retinanah befestigt. Über diese Spule 6 als Teil von IGF 4 und mindestens einer korrespondierenden Spule 7 als Teil von AGF 8 innerhalb der Augenhöhle wird durch die Sklera 9 induktiv eine Kommunikationsverbindung hergestellt. Der Signalweg verläuft durch die Sklera 9 des Auges innerhalb der durch die Conjunctiva 19 begrenzten Augenhöhle verläuft, bleibt der optische Weg von der Linse zu der Retina außerhalb des fovealen Bereich frei. Die Trennbarkeit des Signalweges ermöglicht die separate Implantierbarkeit der innerhalb des Auges implantierten Komponente und der außerhalb des Auges in der Augenhöhle implantierten Komponente.

In der Figur 2 ist eine weitere Realisierung für ein Retinaimplantat veranschaulicht. Hierbei wird die transsklerale Verbindung zwischen IGF 4 und AGF 8 galvanisch über ein Mikrokabel 10 hergestellt, wobei dieses Mikrokabel während der Implantation im Bereich einer Steckverbindung 11 mechanisch getrennt ist und anschließend die Verbindung hergestellt wird. Eine derartige nachträglich herstellbare Mikrokabelverbindung 10 zwischen der z.B. nur die Retinastimulation bewirkenden IGF 4 und einer implantierten, aber außerhalb des Auges befindlichen, Decodier- und/oder Demultiplexing-Funktionen übernehmenden AGF 8 kann bevorzugt so ausgestaltet werden, dass eine geeignete transsklerale Kanüle 12 dauerhaft implantiert wird, durch welche das Mikrokabel 10 geführt und anschließend die Durchführung versiegelt wird. Ferner kann die nachträgliche Mikrokabelverbindung 10 so ausgeführt sein, dass an den beiden galvanisch zu verbindenden Enden (entsprechend Stecker oder Kupplung) eine gleiche Anzahl komplementär geformter Metallkontakte (am einen Kabelende z. B. als Stifte und am anderen Kabelende als Buchsen) vorhanden ist, die während der separaten Implantation von IGF 4 und AGF 8 mit einer isolierenden dünnen Kunststoffmembran zum Schutz vor Flüssigkeitseinwirkung abgedeckt sind.

In einer weiteren Ausführung wird die Mikrokabelverbindung dadurch ausgeführt, dass Stiftteil 11 und Buchsenteil 11' flach einander gegenüber unter Abgleichung der Fluchtlinien der Stifte und korrespondierenden Buchsen positioniert werden und dass anschließend Stiftteil und Buchsenteil sanft so gegeneinander gepresst werden, dass einerseits die isolierenden Membranen durchbrochen und andererseits auch unter Feuchtraumbedingungen sicher isolierte galvanische Verbindungen der korrespondierenden Mikrokabelleitungen 10 hergestellt werden.

In einer weiteren Ausführung kann diese Mikrokabelverbindung nachträglich durch ein geeignetes Trennwerkzeug wieder getrennt werden.

Diese aus Stiftteil 11 und Buchsenteil 11' bestehende lösbare Mikrokabelverbindung kann sowohl außerhalb des Augapfels in der Augenhöhle (siehe Fig. 2) als auch innerhalb der Glaskörperkammer (ohne Abbildung) realisiert werden.

In ebenfalls vorteilhaften Ausführungen wird die drahtlose transsklerale Kommunikation optoelektronisch oder durch Ultraschallsender- und Empfängerpaare auf beiden Seiten eines umschriebenen Sklerabezirks realisiert.

In einer weiteren Ausführung wird in der Wand der Sklera 9 eine Kanüle 12 angeordnet, die mittels einer durchlässigen Membran nach Art eines geschlossenen Fensters abgeschlossen wird.

In einer weiteren Realisierung ist AGF 8 skleral an der Bulbusaußenwand gemäß dem ophthalmologischen Stand der Technik (kleben, heften oder nähen) befestigt und verfügt neben einem Mikrokabel 5 und mindestens einer skleral gegenüber der jeweils korrespondierenden Spule 6 in der Glaskörperkammer befestigten Primärspule 7 über ein weiteres Mikrokabel 15 zur Verbindung mit der außerhalb des Körpers befindlichen Funktionseinheit (EFP) 2.

In einer bevorzugten Ausführung erfolgt die Verbindung zwischen AGF 8 in der Augenhöhle und der externen EPF 2 induktiv über ein Spulenpaar 16, 17 und zugehörige Sende- und Empfangselektronik 18, wobei dieses Spulenpaar 16, 17 durch eine Hautregion im Kopfbereich (z. B. im Bereich der Stirn) getrennt wird und wobei das Mikrokabel 15 von AGF 8 zu Sekundärspule 16 und Empfangselektronik 18 dieser bevorzugten transkutanen induktiven Verbindung nach dem chirurgischen Stand der Technik unter der Haut verlegt wird.

In einer weiteren möglichen Realisierung erfolgt die Verbindung zwischen AGF 8 in der Augenhöhle und der externen EPF 2 über eine geeignete Katheter- und/oder Kabelstruktur (nicht dargestellt).

In einer vorteilhaften Ausführung wird zum Zweck der Funktionseinstellung außerhalb des normalen Implantatbetriebs eine optische und/oder optoelektronische Kommunikation zwischen einer außerhalb des Körpers befindlichen Funktionseinheit und der IGF 4 in der Glaskörperkammer hergestellt.

Die externe Funktionseinheit 2, die einen Encoder sowie Kameramittel umfassen kann, kann bei einer erfindungsgemäßen Ausführungsform auch auf andere Weise vom Patienten getragen werden als mit der herkömmlichen brillenähnlichen Anordnung. So können die erforderlichen Komponenten auch in einer Mütze oder einem Stirnband angeordnet werden, die das Tragen einer möglicherweise unangenehm schweren Brille vermeidbar machen. Dies ermöglicht weiter die Verwendung größerer Komponenten, die für die Verarbeitung einer größeren Anzahl von optischen Kanälen oder Pixeln geeignet sind.

## Patentansprüche

1. Retina-Implantat mit einer ersten intern innerhalb der Glaskörperkammer eines Auges positionierbaren Funktionseinheit (4) und wenigstens einer dritten extern außerhalb des Auges positionierbaren Funktionseinheit (2)
**dadurch gekennzeichnet, dass**
eine zweite Funktionseinheit (8) außerhalb der Glaskörperkammer des Auge und innerhalb der Augenhöhle positionierbar ist sowie ein Signalweg (5, 6, 7) zur Übertragung von Signalen oder Daten zwischen der ersten (4) und zweiten (8) sowie zwischen der zweiten (8) und dritten Funktionseinheiten (2) vorgesehen ist, wobei der Signalweg zwischen der ersten Funktionseinheit (4) und der zweiten Funktionseinheit (8) eine drahtlose induktive Kommunikationsverbindung (6, 7) aufweist.

2. Implantat nach Anspruch 1, **dadurch gekennzeichnet, dass** die drahtlose induktive Verbindung (6, 7) jeweils ein innerhalb des Auges positionierbares Sende- und Empfangselement (6) und ein außerhalb des Auges positionierbares Sende- und Empfangselement (7) aufweist.

3. Implantat nach Anspruch 1 oder Anspruch 2, **dadurch gekennzeichnet, dass** die außerhalb des Auges in der Augenhöhle implantierbare zweite Funktionseinheit (8) zur Signalverarbeitung vorgesehen ist.

## Claims

1. A retinal implant comprising at least one first internal functional unit (4) arranged for being disposed inside the vitreous cavity of an eye and at least one third external functional unit (2) arranged for being disposed outside the eye,
**characterized by**
at least one second external functional unit (8) arranged for being disposed outside the vitreous cavity of the eye and inside the eye orbit and by a signal path (5, 6, 7) for signal and data transmission between the first (4) and the second (8) as well as between the second (8) and the third (2) functional units, wherein the signal path between the first (4) and second (8) functional units has a wireless inductive connection (6, 7).

2. Implant according to claim 1, **characterized in that** the wireless connection (6, 7) comprises each of a sending and receiving element (6) arranged for being disposed within an eye and a sending and receiving element (7) arranged for being disposed outside an eye.

3. Implant according to claim 1 or 2, **characterized in that** the second functional unit (8) arranged for being implanted in the eye orbit outside the eye is provided for signal processing.

## Revendications

1. Implant rétinien comportant une première unité fonctionnelle (4) pouvant être positionnée au niveau interne à l'intérieur de la chambre de corps vitré d'un oeil et au moins une troisième unité fonctionnelle (2) pouvant être positionnée au niveau externe à l'extérieur de l'oeil
caractérisé en ce
qu'une deuxième unité fonctionnelle (8) peut être positionnée à l'extérieur de la chambre de corps vitré de l'oeil et à l'intérieur de l'orbite oculaire et une voie de signal (5, 6, 7) est prévue pour la transmission de signaux ou de données entre les première (4) et deuxième (8) unités fonctionnelles et entre les deuxième (8) et troisième unités fonctionnelles (2), dans lequel la voie de signal présente, entre la première unité fonctionnelle (4) et la deuxième unité fonctionnelle (8), une liaison de communication sans fil inductive (6, 7).

2. Implant selon la revendication 1, **caractérisé en ce que** la liaison inductive sans fil (6, 7) présente respectivement un élément d'envoi et de réception (6) pouvant être positionné à l'intérieur de l'oeil et un élément d'envoi et de réception (7) pouvant être positionné à l'extérieur de l'oeil.

3. Implant selon la revendication 1 ou la revendication 2, **caractérisé en ce que** la deuxième unité fonctionnelle (8) pouvant être implantée à l'extérieur de l'oeil dans l'orbite oculaire est prévue pour le traitement du signal.
